# EUROPEAN PATENT SPECIFICATION

(11) **EP 2 285 289 B1**
(45) Date of publication and mention of the grant of the patent: **29.08.2018**
(21) Application number: 09762568.5
(22) Date of filing: 10.06.2009
(51) Int. Cl.: A61B 8/08, G06T 7/00, G06T 5/00

(54) **ULTRASONIC APPARATUS AND CONTROL METHOD THEREFOR**
ULTRASCHALLVORRICHTUNG UND STEUERUNGSVERFAHREN DAFÜR
APPAREIL À ULTRASONS ET PROCÉDÉ DE COMMANDE ASSOCIÉ

(30) Priority: 13.06.2008 JP 2008155105; 01.04.2009 JP 2009089092
(43) Date of publication of application: 23.02.2011
(73) Proprietor: Canon Kabushiki Kaisha, Tokyo 146-8501 (JP)
(72) Inventor: TATEYAMA, Jiro, Tokyo (JP)
(74) Representative: TBK
(86) International application number: PCT/JP2009/060972
(87) International publication number: WO 2009/151142

(56) References cited:
- WO-A1-2004/054447
- US-A1- 2005 228 254
- DURIC NEBOJSA; LITTRUP PETER; BABKIN ALEX; CHAMBERS DAVID; AZEVEDO STEPHEN; KALININ ARKADY; PEVZNER ROMAN; TOKAREV MIKHAIL; HOLSAP: "Development of ultrasound tomography for breast imaging: Technical assessment" MEDICAL PHYSICS, AIP, MELVILLE, NY, US, vol. 32, no. 5, 21 April 2005 (2005-04-21) , pages 1375-1386, XP012075338 ISSN: 0094-2405
- RIGBY K W; CHALEK C L; HAIDER B H; LEWANDOWSKI R S; O'DONNELL M; SMITH L S; WILDES D G: "Improved in vivo abdominal image quality using real-time estimation and correction of wavefront arrival time errors" ULTRASONICS SYMPOSIUM, 2000 IEEE OCT 22-25, 2000, 20001022 - 20001025 PISCATAWAY, NJ, USA,IEEE, vol. 2, 22 October 2000 (2000-10-22), pages 1645-1653, XP010540930
- NAPOLITANO D; CHOU C H; MCLAUGHLIN G; JI T L; MO L; DEBUSSCHERE D; STEINS R: "Sound speed correction in ultrasound imaging" ULTRASONICS, IPC SCIENCE AND TECHNOLOGY PRESS LTD. GUILDFORD, GB, vol. 44, 22 December 2006 (2006-12-22), pages E43-E46, XP025009137 ISSN: 0041-624X [retrieved on 2006-12-22]
- MALLART R; FINK M ED - ERMERT H; HARJES H-P: "SOUND SPEED FLUCTUATIONS IN MEDICAL ULTRASOUND IMAGING COMPARISON BETWEEN DIFFERENT CORRECTION ALGORITHMS" ACOUSTICAL IMAGING. BOCHUM, APR. 3 - 5, 1991; 19910403 NEW YORK, PLENUM PRESS, US, vol. 19, 3 April 1991 (1991-04-03), pages 213-218, XP000333576

## Description

### Technical Field

The present invention relates to a technique for imaging the interior of an object to be inspected by using ultrasonic waves.

### Background Art

As an ultrasonic apparatus for imaging the interior of an object (specimen) to be inspected by using ultrasonic waves, there has been known an ultrasonic diagnostic apparatus for use in medical diagnosis, for example. In the ultrasonic apparatus, an ultrasonic probe is used which includes a plurality of ultrasonic transducers having the function of transmitting and receiving ultrasonic waves. When an ultrasonic beam formed in combination of a plurality of ultrasonic waves is transmitted from the ultrasonic probe to the specimen, the ultrasonic beam is reflected in a region of different acoustic impedance (i.e., a boundary of a tissue) in the interior of the specimen. The appearance in the interior of the specimen can be reproduced on a screen by receiving an ultrasonic wave echo generated in this manner and constructing an image based on the intensity or magnitude of the ultrasonic wave echo.

In such an ultrasonic image, it has conventionally been attempted to extract a contour (boundary) of the tissue in an accurate manner. This is because it is possible to make use of the contour thus extracted for three-dimensional image processing or diagnosis such as distinction of benignancy and malignancy of tumor or the like.

As a related art, Japanese patent application laid-open No. H07(1995)-194597 describes a method of using threshold processing as a method of automatically extracting the outline or contour of a desired specimen region. A luminance value of an ultrasonic image is searched for along a received sound wave ray, and when a predetermined number or more of luminance values, each equal to or higher than a threshold, have been continuously searched, processing with a predetermined threshold or extraction of a boundary position is performed on the pixels on the received sound wave ray. In addition, as another related art, there is a method that is disclosed in Japanese patent application laid-open No. 2004-181240.
Prior art which is related to this field of technology can be found e.g. in document WO 2004/054447 A1 disclosing an ultrasonic apparatus for estimating artery parameters, in non-patent literature DURIC NEBOJSA; LITTRUP PETER; BABKIN ALEX; CHAMBERS DAVID; AZEVEDO STEPHEN; KALININ ARKADY; PEVZNER ROMAN; TOKAREV MIKHAIL; HOLSAP: "Development of ultrasound tomography for breast imaging: Technical assessment", MEDICAL PHYSICS, AIP, MELVILLE, NY, US, vol. 32, no. 5, 21 April 2005 (2005-04-21), pages 1375-1386, in non-patent literature RIGBY K W; CHALEK C L; HAIDER B H; LEWANDOWSKI R S; O'DONNELL M; SMITH L S; WILDES D G: "Improved in vivo abdominal image quality using real-time estimation and correction of wavefront arrival time errors" ULTRASONICS SYMPOSIUM, 2000 IEEE OCT 22-25, 2000, 20001022-20001025 PISCATAWAY, NJ, USA, IEEE, vol. 2, 22 October 2000 (2000-10-22), pages 1645-1653, and in document US 2005/0228254 A1 disclosing a method and apparatus for detecting anatomic structures. The afore-mentioned document WO 2004/054447 A1 discloses an apparatus and a method according to the preambles of claims 1 and 9.

### Summary of Invention

In case where an operator (inspector) such as an inspecting engineer, etc., performs image diagnosis by using an ultrasonic diagnostic apparatus, it is the general use of the apparatus that the operator applies the search unit to a diagnostic region of the specimen while looking at the ultrasonic image, and moves the position and the angle of the search unit so as to find an internal organ in the form of an object to be inspected. In the ultrasonic image at that time, the position and the shape of the object change dynamically. In order to obtain accurate contour information from such a time-varying or dynamic image in real time, it is necessary to execute boundary extraction processing for each frame.

However, the conventional boundary extraction technique involves a problem that it is difficult to achieve such real-time processing. For example, the technique of the first patent document is to extract the outline or contour of an object to be inspected by performing threshold processing on entire sound ray data in an exhaustive manner by comparing the sound ray data with a threshold value and determining a boundary position. In such a technique, the amount of calculation becomes huge (though it depends on the processing power of the apparatus), processing of all the frames in real time is not practical. Therefore, to apply such a conventional technique to dynamic image processing could not help reducing the frame rate of the dynamic image.

The present invention has been made in view of the above-mentioned circumstances, and has for its object to provide a technique that enables an accurate contour (boundary) to be extracted in real time from an ultrasonic wave dynamic image in motion.

The present invention provides an ultrasonic apparatus according to claim 1 and a control method for an ultrasonic apparatus according to claim 9.

According to the present invention, an accurate contour (boundary) can be extracted in real time from an ultrasonic wave dynamic image in motion. Accordingly for example, it is possible to dynamically extract and observe a boundary of a diagnostic region while moving an ultrasonic probe.

Further features of the present invention will become apparent from the following description of exemplary embodiments with reference to the attached drawings.

### Brief Description of Drawings

Fig. 1A through Fig. 1C are block diagrams illustrating the construction of an ultrasonic diagnostic apparatus.
Fig. 2A is a view illustrating canning of an ultrasonic beam.
Fig. 2B is a view illustrating a received waveform of ultrasonic wave echo.
Fig. 3A through Fig. 3C are flow charts of boundary extraction processing.
Fig. 4A and Fig. 4B are views explaining a method of setting a search region.
Fig. 5A through Fig. 5D are views explaining the processing of tracking a boundary line.
Fig. 6A through Fig. 6C are views explaining a second embodiment.

### Description of Embodiments

Hereinafter, preferred embodiments of the present invention will be described in detail by way of example while referring to the accompanying drawings. Here, note that although a medical ultrasonic diagnostic apparatus is shown herein as one example of an ultrasonic apparatus, the present invention is preferably applicable to a variety of kinds of ultrasonic inspection apparatuses in which objects other than a living body are made the objects to be inspected.

### <FIRST EMBODIMENT>

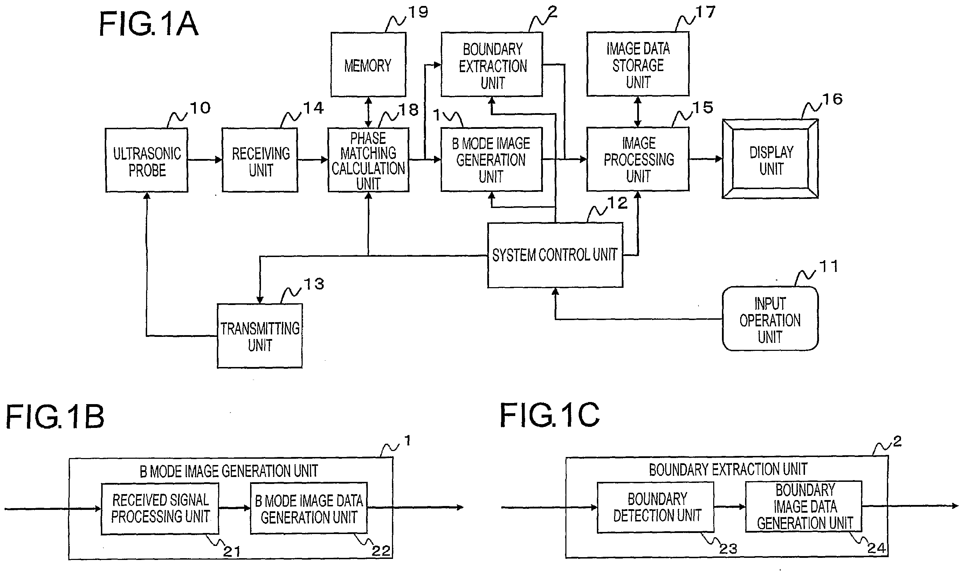

Fig. 1A is a block diagram illustrating the construction of an ultrasonic diagnostic apparatus according to a first embodiment of the present invention. The ultrasonic diagnostic apparatus according to this embodiment is provided with a function (boundary extraction unit 2) to extract a boundary between different tissues (between media) in addition to a B mode image generation function (B mode image generation unit 1) which is possessed by a general ultrasonic diagnostic apparatus.

As illustrated in Fig. 1A, the ultrasonic diagnostic apparatus according to this embodiment includes an ultrasonic probe 10, an input operation unit 11, a system control unit 12, a transmitting unit 13, a receiving unit 14, an image processing unit 15, a display unit 16, and an image data storage unit 17. In addition, the ultrasonic diagnostic apparatus is further provided with a phase matching calculation unit 18 and a memory 19.

### (Ultrasonic probe)

The ultrasonic probe 10 is used so as to be placed in contact with a specimen, so that it transmits and receives ultrasonic beams toward and from the specimen. The ultrasonic probe 10 is provided with a plurality of ultrasonic transducers (ultrasonic vibrators). An ultrasonic transducer is an element that transmits an ultrasonic beam based on a drive signal applied thereto, receives an ultrasonic wave echo (reflection wave) and outputs an electric signal corresponding to the intensity of reflection thereof. These ultrasonic transducers are arranged in a one-dimensional or two-dimensional manner thereby to form a transducer array.

The ultrasonic transducers are each composed of a transducer which has electrodes formed at opposite ends of a material (piezoelectric material) having piezoelectricity. As such a piezoelectric material, there are used, for example, piezoelectric ceramic represented by PZT (Pb (lead) zirconate titanate), polymer piezoelectric element represented by PVDF (polyvinylidene difluoride), and so on. The piezoelectric material is caused to expand and contract when a pulsed electric signal or a continuous wave electric signal is sent to the electrodes of such a transducer thereby to apply a voltage thereto. In accordance with the expansion and contraction, a pulse-shaped ultrasonic wave or a continuous-wave ultrasonic wave is generated from each transducer, so that an ultrasonic beam is formed by the combination of these ultrasonic waves. In addition, the individual transducers are caused to expand and contract to generate electric signals, respectively, by receiving the transmitted ultrasonic waves. These electric signals are output as detection signals for the corresponding ultrasonic waves.

Alternatively, a plurality of kinds of elements of different transducing schemes can be used as the ultrasonic transducers. For example, the above-mentioned transducers are used as elements for transmitting ultrasonic waves, and ultrasonic transducers of photodetection type are used as elements for receiving ultrasonic waves. The ultrasonic transducer of photodetection type is one that makes detection by converting an ultrasonic beam into an optical signal, and is composed of, for example, a Fabry-Perot resonator or a Fiber Bragg grating.

### (Drive Unit)

The transmitting unit 13 is a circuit that supplies a drive signal to the respective ultrasonic transducers so as to transmit an ultrasonic wave from the ultrasonic probe 10. The transmitting unit 13 is composed of a plurality of drive circuits corresponding to the individual ultrasonic transducers, respectively.

The receiving unit 14 is a circuit that processes the ultrasonic waves (reflection waves) received by the ultrasonic probe 10. The receiving unit 14 is also composed of a plurality of receiving circuits corresponding to the individual ultrasonic transducers, respectively. The receiving circuits apply analog amplification processing to the detection signals output from the ultrasonic transducers by the use of preamplifiers and TGC (time gain compensation) amplifiers, respectively. The levels of the detection signals are adjusted to the input signal levels of the A/D converters, respectively, by the analog amplification processing. The analog signals output from the TGC amplifiers are converted into detected data in the form of digital signals by means of the A/D converters. A plurality of pieces of detected data (received signals) corresponding to the individual ultrasonic transducers, respectively, are output from the receiving unit 14.

### (Other Construction of the Apparatus)

The memory 19 includes a plurality of line memories corresponding to a plurality of the receiving circuits, respectively, and stores the detected data output from the individual receiving circuits in a time series manner.

The phase matching calculation unit 18 performs calculation processing, i.e., reception focus processing so as to adjust the phase of the detected data. The phase matching calculation unit 18 provides delays corresponding to the focal position to the plurality of pieces of detected data stored in the memory 19, respectively, and thereafter adds them to one another. As a result, sound ray data representing ultrasonic wave information along desired scanning lines is generated. The phase matching calculation unit 18 is constituted by a shift register delay line, a digital minute delay machine, aCPU (central processing unit) with software, or any combination of these.

The input operation unit 11 is used when an operator inputs instructions and information to the ultrasonic diagnostic apparatus. The input operation unit 11 includes a keyboard, an adjustment knob, and a pointing device such as a mouse, etc.

The system control unit 12 is constituted by a processor and software, and controls respective parts of the ultrasonic diagnostic apparatus based on the instructions and information input from the input operation unit 11.

### (Ultrasonic B Mode Image)

Fig. 1B is a view illustrating the internal configuration of the B mode image generation unit 1. The B mode image generation unit 1 is a function to generate ultrasonic images from the received signals, and is composed of a received signal processing unit 21 and a B mode image data generation unit 22. The received signal processing unit 21 applies envelope detection processing and STC (sensitivity time gain control) to the sound ray data produced by the phase matching calculation unit 18, and the B mode image data generation unit 22 generates and outputs B mode image data.

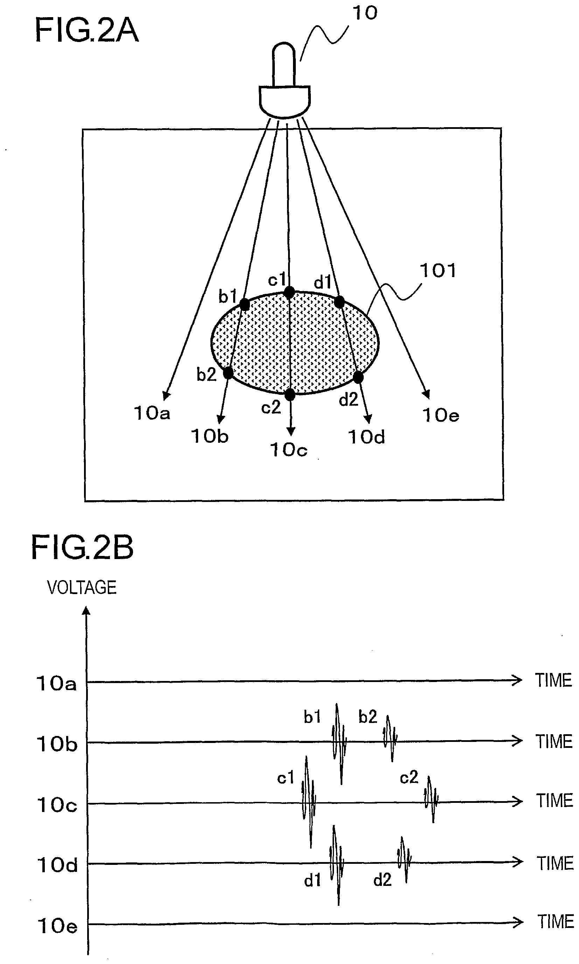

Fig. 2A is a view in which ultrasonic wave echoes reflected on a surface of a reflector 101 are received when scanning lines 10a through 10e are sequentially sent and scanned toward the reflector 101 by using the ultrasonic probe 10 including the ultrasonic transducers. In case where a sector type search unit is used for the ultrasonic probe 10, beam steering can be arbitrarily controlled, so the direction in which the ultrasonic beam is scanned can be freely switched over. In addition, in the case of a convex search unit, a transducer array is arranged in a sector shape, so the ultrasonic beam will be scanned in the sector shape decided by the construction of the transducer array. At this time, a strong ultrasonic wave echo is returned on the surface of the reflector 101, i.e., at a location which becomes the boundary (contour) of a medium existing in the specimen.

Fig. 2B illustrates the received waveforms of the ultrasonic wave echoes in the individual scanning lines 10a through 10e. The axis of abscissa indicates time, and the axis of ordinate indicates the voltages of the received signals. As illustrated in Fig. 2A, those places on the surface of the reflector 101 from which strong ultrasonic wave echoes are returned are a boundary position b1 and a boundary position b2 on the scanning line 10b, a boundary position c1 and a boundary position c2 on the scanning line 10c, and a boundary position d1 and a boundary position d2 on the scanning line 10d, respectively. At this time, signals reflected on the boundary of reflector 101 are observed as the received waveforms of the scanning lines 10b through 10d, as illustrated in Fig. 2B. Specifically, points of high amplitude appear at positions of b1 and b2, respectively, on the scanning line 10b, and such points appear at positions of c1 and c2, respectively, on the scanning line 10c, and at positions of d1 and d2, respectively, on the scanning line 10d. Thus, it becomes possible to extract the boundary of the reflector 101 by connecting these points of high amplitude with one another. Here, note that in an actual apparatus, it is necessary to extract a smoother boundary shape by making the scanning angle of the ultrasonic beam (resolving power) much finer.

Fig. 1C is a view illustrating the internal construction of the boundary extraction unit 2. The boundary extraction unit 2 is composed of a boundary detection unit 23 and a boundary image data generation unit 24. The boundary detection unit 23 detects the boundary (contour) of the medium existing in the specimen based on the signal intensity or magnitude of the sound ray data produced by the phase matching calculation unit 18. The boundary image data generation unit 24 generates boundary image data by allocating a predetermined color to a region (display region) on a display screen corresponding to the boundary detected by the boundary detection unit 23. The principle and operation of the boundary extraction performed in the boundary extraction unit 2 will be described later in detail.

The image processing unit 15 generates synthetic image data in which a boundary image is overlapped on a region of B mode image, based on the B mode image data generated by the B mode image data generation unit 22 and the boundary image data generated by the boundary image data generation unit 24. The region of the B mode image in which the boundary image is overlapped can be automatically decided by the image processing unit 15, or can be manually designated by the operator with the use of the input operation unit 11.

The image data storage unit 17 stores the synthetic image data thus generated. In addition, the image processing unit 15 generates image data for screen display by applying predetermined image processing including scan conversion, gradation processing, etc., to the synthetic image data. The display unit 16 includes a display unit such as a CRT, an LCD, etc., and an ultrasonic image is displayed based on the image data to which the image processing has been applied by the image processing unit 15.

### (Boundary Extraction Processing)

Next, reference will be made to a processing flow of the principle and operation of the boundary extraction in this embodiment. However, the processing to be described herein is merely one specific example, and the scope of the present invention should not be limited to this.

In general, it is difficult to accurately extract only a target boundary line (contour) from an ultrasonic image. In addition, as stated above, if boundary detection processing is applied to all the sound ray data in the image in an exhaustive manner, the amount of data to be calculated becomes huge, and hence real-time processing thereof also becomes difficult. Accordingly, in this embodiment, the above-mentioned problem is solved as follows. That is, (1) after a boundary line at the first time (an initial boundary line) is decided by using the teaching or instruction of the operator, (2) the processing of searching and tracking the boundary line is performed on a target region in the form of a nearby region including the boundary line.

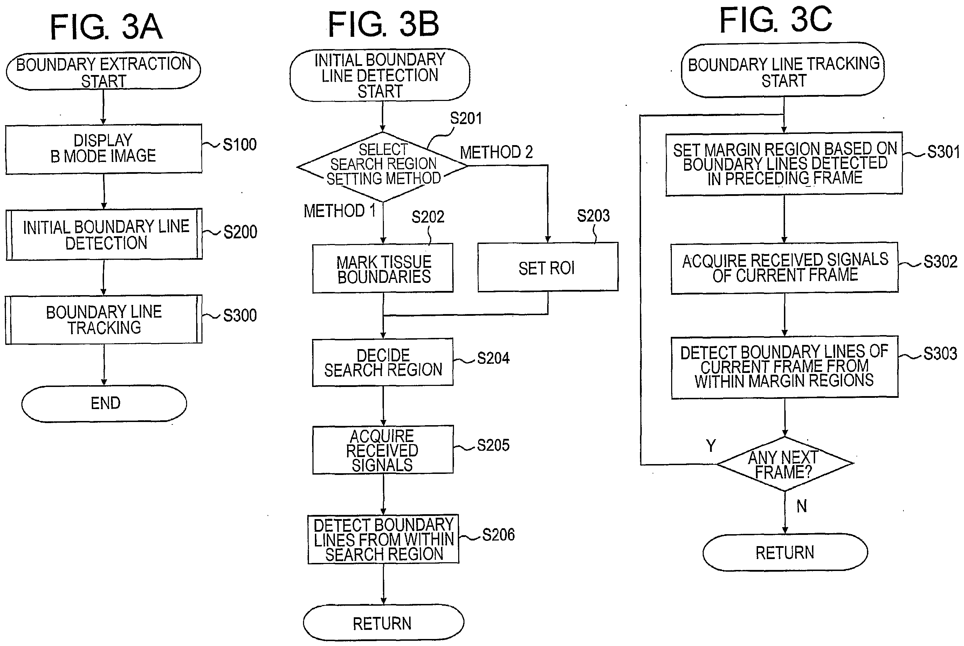

Now, reference will be made to the flow of the boundary extraction processing by using the flow charts of Fig. 3A, Fig. 3B and Fig. 3C. Fig. 3A illustrates the main flow of the boundary extraction processing, Fig. 3B illustrates the flow of the detection processing of the initial boundary line, and Fig. 3C illustrates the flow of the boundary line tracking processing. Here, note that the processing illustrated in Fig. 3A through Fig. 3C is mainly executed by the boundary extraction unit 2, but includes the processing achieved by its cooperation with the B mode image generation unit 1, the system control unit 12, the input operation unit 11, the image processing unit 15, the display unit 16, and so on, too.

As shown in Fig. 3A, when the boundary line extraction processing starts, the B mode image is displayed on the display unit 16 (S100), and the detection processing of the initial boundary line is execute (S200). Here, note that when the operator moves the ultrasonic probe, the B mode image (ultrasonic image) is sequentially generated at a predetermined period (at each position of the ultrasonic probe in the object to be inspected), so that time series ultrasonic images are thus obtained. The initial boundary line detection processing is executed on the first ultrasonic image in such time series ultrasonic images.

### (Detection of Initial Boundary Line)

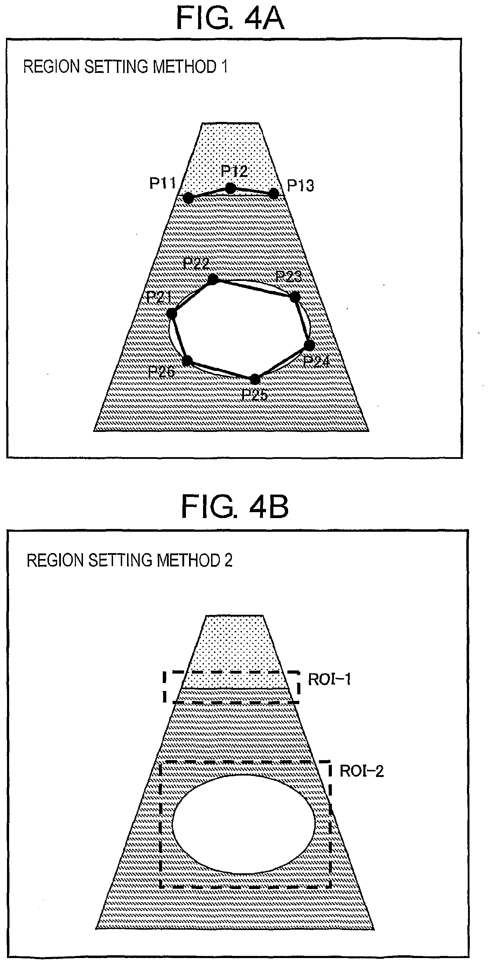

As illustrated in Fig. 3B, first of all, the operator sets a search region for the initial boundary line (S201). In this embodiment, a method of setting the search region can be selected from among two kinds of methods, i.e., method 1 (Fig. 4A: a tissue boundary marking method) and method 2 (Fig. 4B: an ROI setting method).

The tissue boundary marking method according to method 1 is a technique that the operator teaches or instructs the position of the boundary line, as illustrated in Fig. 4A. The operator marks boundary candidate points on the B mode image displayed on the display unit 16 by using the input operation unit 11 (e.g., a pointing device) (S202). In the example of Fig. 4A, points P11 through P13 are marked in the part of a lamellar tissue boundary, and points P21 through P26 are marked in the part of a circular tissue boundary. For instance, a boundary between a fat layer and a soft tissue or the like is assumed as the lamellar tissue boundary. Also, for instance, a contour of an internal organ, a tumor or the like is assumed as the circular tissue boundary. When the boundary candidate points are input in this manner, the detection of the initial boundary lines is performed based on those point sequences. Specifically, a region of a predetermined range around each of those boundary candidate points, or a region of a predetermined width around a line connecting between a sequence of points, is set as a search region of an initial boundary line (S204).

The ROI setting method according to method 2 is a technique in which the operator teaches or instructs, as an ROI (area of interest), a region where a boundary line exists, as illustrated in Fig. 4B. The operator can set the ROI by designating, as an area, a part of the image displayed on the display unit 16 by using the input operation unit 11 (S203). In the example of Fig. 4B, an ROI -1 is designated in the part of the lamellar tissue boundary, and an ROI-2 is designated in the part of the circular tissue boundary. These ROIs are set as the search regions of initial boundary lines (S204).

After the search regions have thus been fixedly set, the boundary extraction unit 2 acquires the received signals (sound ray data) of a current frame from the phase matching calculation unit 18 (S205), and detects boundary lines only from those received signals which correspond to the search regions (S206). As a result, the boundary lines can be detected in a shorter period of time than when boundary detection processing is executed over the entire regions of the received signals. Moreover, since the regions where the boundaries exist are taught, false detection of the boundaries can be reduced and the initial boundary lines can be decided in an accurate manner.

The detection processing of the initial boundary lines by means of the boundary extraction unit 2 as referred to above corresponds to an initial boundary setting unit in the present invention that serves to set the initial boundaries for the first ultrasonic image in the time series ultrasonic images. The time series ultrasonic images means a group of temporally continuous ultrasonic images that have been sequentially generated at a predetermined period (frame rate). When the operator moves the ultrasonic probe, an ultrasonic image is sequentially generated at each position of the ultrasonic probe in the object to be inspected. Here, note that it is desirable to fix the position of the ultrasonic probe 10 so as to prevent the boundary positions from changing during the initial boundary line detection processing.

### (Tracking of Boundary Lines)

When the initial boundary lines are decided as stated above, the control flow shifts to the boundary line tracking processing (S300). Here, note that after the tracking processing has been started, it is possible to observe the specimen while moving the position and the angle of the ultrasonic probe 10. Thus, the time series ultrasonic images (ultrasonic wave dynamic images) are sequentially generated at the predetermined frame rate, and the extraction of the boundaries is sequentially performed on the ultrasonic images of each frame by means of a method that will be described below.

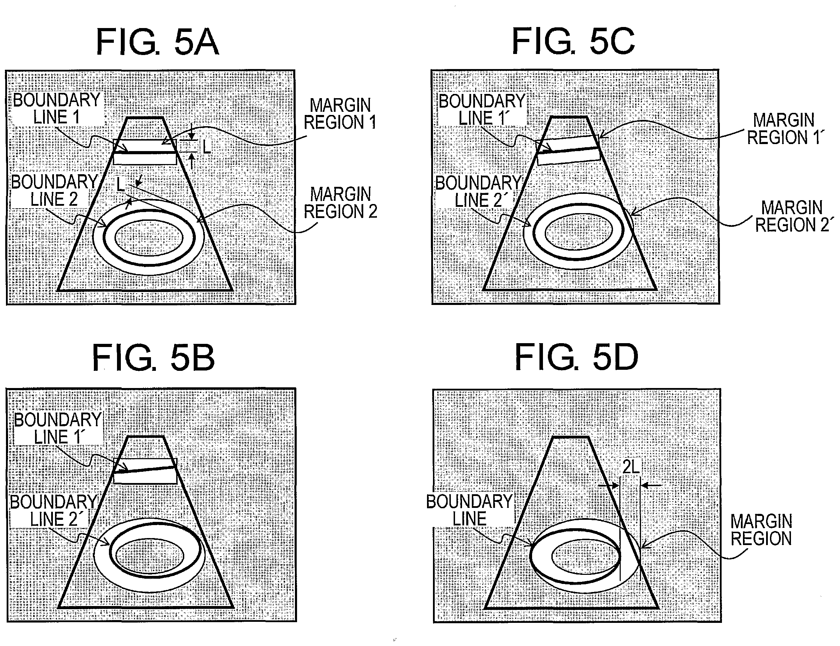

As illustrated in Fig. 3C, the boundary extraction unit 2 first sets margin regions (target regions) based on the initial boundary lines (S301) . Specifically, ranges of a distance L from and around the boundary lines are set as margin regions, as illustrated in Fig. 5A. A rectangular margin region 1 having a width of 2L around a lamellar boundary line 1 is set for the lamellar boundary line 1 such as a fat layer illustrated in Fig. 5A. In addition, a doughnut-shaped margin region 2 having a width of 2L around a circular boundary line 2 is set for the circular boundary line 2 such as the contour of a kidney.

Subsequently, the boundary extraction unit 2 acquires the received signals of the current frame (frame being processed) from the phase matching calculation unit 18 (S302) . The example of an image of the current frame is illustrated in Fig. 5B. When the position and the angle of the ultrasonic probe 10 are changing, the positions of boundary lines 1' and 2' also change in comparison with those of an image of a preceding frame, as illustrated in Fig. 5B. However, the amount of movement of the search unit during a one-frame period of time is limited, and there is some correlation between frames, so there is a high probability that the boundaries 1' and 2' of the current frame are contained in the margin regions of the boundaries of the preceding frame.

Accordingly, the boundary extraction unit 2 executes the boundary line detection processing for only those received signals which correspond to the reflection waves reflected at an inner side of each of the margin regions (S303). As a result, the boundary lines can be detected in a extremely shorter period of time than when boundary detection processing is executed for the entire region of the received signals.

The processing in steps S301 through S303 is repeatedly executed every one frame. In the processing of the following frame, the margin regions 1' and 2' are again set (updated) in such a manner as to contain the boundary lines 1' and 2' extracted in the preceding frame, as illustrated in Fig. 5C (S301), and these margin regions thus set are used for the boundary detection processing of the following frame (S302, S303).

The value of the size L of each margin region can be fixed, or can be changed, or can be caused to vary dynamically. It is desirable that the size (the value of L) and the position of each margin region be decided in consideration of the speed and the direction of movement of the search unit being driven by the operator (based on information of the movement between the positions of the ultrasonic probe) . That is, when an operator causing the search unit to move at fast speed uses the search unit, the margin regions are increased by making the value of L larger. However, the larger the value of L, the larger the search regions become, so the processing time of the boundary detection accordingly increases. Thus, an upper limit should be set for the value of L. When the operator slowly moves the search unit, the search regions can be narrowed by decreasing the value of L. If the detection processing can be shortened, there will be a merit or advantage that high-quality movie (moving image) display becomes possible due to further improved frame rate. In addition, as for the setting of the size L of the margin regions, the operator can manually set the size L by initial setting. Or, the apparatus can also automatically set the size L for each frame by learning the movement operation condition of the search unit and estimating the amount of movement per frame. It is also desirable to estimate (predict) the direction of movement of each boundary line in an image from the history of the direction of movement of the search unit or the history of the tracking processing or the like, and to decide the position of each margin region based on the result of the estimation. Fig. 5D is a view illustrating a set position of a margin region in case where a boundary line is estimated to move to the right side. For example, when it is estimated that the boundary line moves to the right side, a margin region having a size or width of 2L is arranged at a position shifted to the right from the current boundary line. With such an arrangement, the range of movement of the search unit can be covered up to twice that in the case of Fig. 5B even with the same area as that of the margin region illustrated in Fig. 5B. In addition, by providing an acceleration sensor, a speed sensor, a position sensor and so on to the search unit, the direction of movement and the operating speed of the search unit (above-mentioned information of the movement between the positions of the ultrasonic probe) can be detected, and the direction and the size of L can be dynamically changed in accordance with the result of the detection. If going one step further, it becomes possible to set the margin region L to a more effective range by estimating (learning) the pattern of movement operation from the information of a sensor mounted on the search unit.

As described above, in this embodiment, by limiting the search range of a boundary line, an accurate contour (boundary) can be extracted in real time from an ultrasonic dynamic image in motion. Accordingly, for example, it is possible to dynamically extract and observe the boundary of a diagnostic region while moving an ultrasonic probe.

### <SECOND EMBODIMENT>

Next, reference will be made to an ultrasonic diagnostic apparatus according to a second embodiment of the present invention. The ultrasonic diagnostic apparatus of this second embodiment is different from that of the first embodiment in that it has a sound speed setting function to set the value of sound speed for each region delimited by a boundary. The other construction of this second embodiment is similar to that of the first embodiment, and hence in the following, those which are different from the first embodiment will be mainly described.

In a conventional ultrasonic diagnostic apparatus, a delay time in the electronic focus is calculated on the assumption that the speed of sound in a living body is a specific value (in general, 1,530 m/sec or 1,540 m/sec as specified in JIS). However, in actuality, the speed of sound is different according to the kind of tissues (media) . In general, the speed of sound is about 3,000 m/sec in bone, about 1,590 m/sec in muscle, and about 1,470 m/sec in fat.

When an object to be inspected has regions in which the speed of sound is different in this manner, the arrival points in time of the individual received signals do not coincide with one another even if the individual delay times decided on the assumption that the speed of sound is uniform are given to the individual received signals, respectively. Accordingly, even if all these individual received signals are added together, a focused signal is not obtained, thus resulting in a blurred tomographic image. The larger the difference between the actual speed of sound in each tissue and a set reference speed of sound (1,530 m/sec), the larger the amount of focal shift becomes. In addition, the larger the layer thickness of each tissue, the larger the above-mentioned amount of focal shift becomes.

For example, in case where a brain is inspected through a transcranial skull, or where a liver is inspected, or where a thyroid is inspected, an actually focused position shifts to a short distance direction from a focusing position (the position of focusing when assuming that a living body is an ultrasonic wave transmission medium of a uniform speed of sound having a sound speed of 1,530 m/sec). In addition, in case where a mammary gland is inspected, an actually focused position shifts to a long distance direction from the focusing position. Specifically, when acoustic diagnosis of a liver in a abdomen is performed, a fat layer of a sound speed of about 1,470 m/sec exists in the vicinity of a body surface, and a muscle layer of a sound speed of about 1,540 m/sec exists under the fat layer, and further, a liver of a sound speed of about 1,540 m/sec similarly exists under the muscle layer. The thickness of the fat layer varies depending on person, so individual delay amounts for the respective received signals can not be uniformly corrected. In addition, the fat layer is sometimes deposited in the muscle or the liver.

Here, note that the values of the speeds of sound are used not only for the determination of delay times in the received focus but also for the generation of an ultrasonic image, various measurements on the ultrasonic image and so on. When there is a difference between the actual speed of sound in each tissue and the set reference speed of sound, a distortion is generated in the ultrasonic image, and the error of measurements obtained from the ultrasonic image becomes large, so it is undesirable.

In order to solve such a problem, the ultrasonic diagnostic apparatus of this embodiment is provided with a sound speed setting function to set an appropriate value of the speed of sound corresponding to the acoustic characteristic of a tissue in each of the regions delimited by boundaries.

### (Sound Speed Setting Processing)

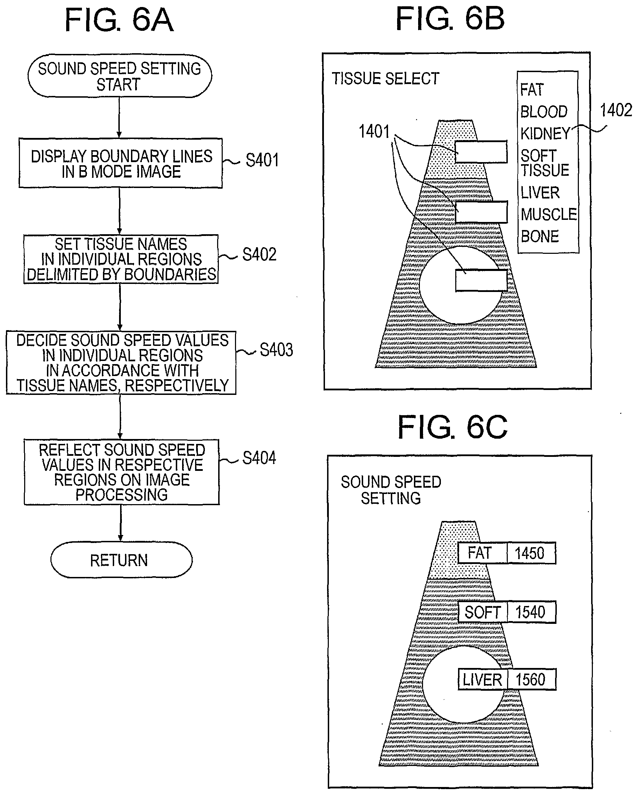

Fig. 6A is a flow chart illustrating the flow of the sound speed setting processing. The sound speed setting processing is mainly executed by a system control unit 12, but includes the processing achieved by its cooperation with a boundary extraction unit 2, a B mode image generation unit 1, an input operation unit 11, an image processing unit 15, a display unit 16, a phase matching calculation unit 18, and so on, too. Here, note that the sound speed setting processing according to the system control unit 12, etc., corresponds to a sound speed setting unit of the present invention.

As shown in Fig. 6A, first of all, an image with a boundary line being overlapped on a B mode image based on the boundary line information obtained by boundary extraction processing (Fig. 3A through Fig. 3C) is displayed on the display unit 16 (S401).

Then, an operator selects and sets a tissue name in each of the regions delimited by boundaries by using the input operation unit 11 (S402). Fig. 6B is a view illustrating one example of a selection screen for tissue names. Boxes 1401 in which names are to be input, respectively, are displayed in the individual regions delimited by boundary lines. Also, a list of tissue names is displayed in a tissue list 1402. By making use of the input operation unit 11 such as a pointing device, the operator can select tissue names from the tissue list 1402, and set them into the individual boxes 1401, respectively. Here, note that provision can be made for a function of determining and setting the tissue names in an automatic manner instead of manually setting the tissue names. As an automatic setting technique, there can be considered, for example, a method of collating and matching tissue names to images of MRIs or X rays, a method of detecting the speed of sound of an ultrasonic wave echo in each tissue by taking autocorrelation for each element of a transducer array, and so on.

Subsequently, the values of the speeds of sound in the individual regions are decided in accordance with the tissue names thus set, respectively (S403). Fig. 6C illustrates one example of a sound speed setting screen. The selected tissue names and the values of the speeds of sound corresponding to the tissues are displayed in the boxes of the individual regions, respectively. In the example of Fig. 6C, it is set sequentially from top to bottom in the following manner; "fat: 1,450 m/sec", "soft tissue: 1,540 m/sec", and "Kidney: 1,560 m/sec".

Thereafter, the values of the speeds of sound in the individual regions are passed from the system control unit 12 to the respective blocks such as the phase matching calculation unit 18, the image processing unit 15 and so on, so that they are reflected in the processing of an ultrasonic image (S404) . Specifically, the phase matching calculation unit 18 calculates (corrects) the delay times of the individual ultrasonic transducers by using the values of the speeds of sound in the individual regions, respectively. As a result, the shift or deviation in position of the electronic focus is improved, thereby suppressing the blurring or defocusing of the image as well as the reduction of the signal level. Further, the image processing unit 15 corrects the distortion of the ultrasonic image by using the values of the speeds of sound in the individual regions. By adopting appropriate values of the speeds of sound in the individual regions, respectively, the degradation of image quality due to the non-uniformity of the speeds of sound in the living body can be suitably corrected in this manner.

The technique of this embodiment is effective for the diagnosis of an object to be inspected in which there exist a lot of tissues of different speeds of sound, which frequently appears in ordinary diagnosis. The technique of this embodiment can also improve the image quality degradation and the image distortion of an ultrasonic image in such a case, and decrease measurement errors on the image.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

1. An ultrasonic apparatus comprising:
an ultrasonic probe (10) that transmits ultrasonic waves to an interior of an object to be inspected, and receives reflection waves thereof;
a receiving unit (14) that outputs received signals based on the reflection waves received by the ultrasonic probe;
an image generation unit (15, 24) that produces ultrasonic images from the received signals;
a boundary extraction unit (2) that extracts boundaries between media appearing in each of the ultrasonic images based on the intensity of each of the received signals;
a display unit (16); and
wherein the boundary extraction unit performs boundary extraction processing on individual time series ultrasonic images sequentially generated,
**characterized in that** the boundary extraction unit
sets a respective target region in a part of each ultrasonic image being processed so as to include boundaries extracted in an ultrasonic image preceding the ultrasonic image being processed, and
performs boundary extraction processing in the ultrasonic image being processed by using received signals corresponding to reflection waves reflected at an inner side of the target region thus set.

2. The ultrasonic apparatus according to claim 1, wherein
the time series ultrasonic images are respectively generated at individual positions of the ultrasonic probe on the object to be inspected; and
the target region is set in a part of the ultrasonic image being processed based on information of movement between the positions of the ultrasonic probe.

3. The ultrasonic apparatus according to claim 1, further comprising:
an initial boundary setting unit that sets an initial boundary for a first ultrasonic image in the time series ultrasonic images.

4. The ultrasonic apparatus according to claim 3, wherein
the display unit displays the ultrasonic image produced by the image generation unit, and
the initial boundary setting unit detects the initial boundary either based on boundary candidate points marked on the first ultrasonic image displayed on the display unit, or by using a region of interest selected on the first ultrasonic image displayed on the display unit.

5. The ultrasonic apparatus according to claim 1, further comprising:
a sound speed setting unit that sets a value of a speed of sound in each of regions delimited by the boundaries.

6. The ultrasonic apparatus according to claim 5, wherein
the sound speed setting unit decides the value of the speed of sound in each region in accordance with a tissue name set in each of the regions delimited by the boundaries.

7. The ultrasonic apparatus according to claim 5, further comprising:
an image processing unit that corrects the ultrasonic image by using the value of the speed of sound in each region set by the sound speed setting unit.

8. The ultrasonic apparatus according to claim 5, wherein
the ultrasonic probe has a plurality of ultrasonic transducers;
the apparatus includes a phase matching calculation unit that adds the received signals together after giving delays corresponding to a focal position to the received signals of the individual ultrasonic transducers, respectively; and
the phase matching calculation unit calculates a delay time of each ultrasonic transducer by using the value of the speed of sound in each region set by the sound speed setting unit.

9. A control method for an ultrasonic apparatus which includes an ultrasonic probe (10) that transmits ultrasonic waves to an interior of an object to be inspected, and receives reflection waves thereof, a receiving unit (14) that outputs received signals based on the reflection waves received by the ultrasonic probe, an image generation unit (15, 24) that generates ultrasonic images from the received signals, and a boundary extraction unit (2) that extracts boundaries between media appearing in each of the ultrasonic images based on the intensity of each of the received signals, and a display unit (16),
the control method for an ultrasonic apparatus comprising:
a step of sequentially generating time series ultrasonic images from the received signals; and
a step of sequentially extracting boundaries with respect to the ultrasonic images thus produced;
**characterized in that** the step of extracting the boundaries comprises:
a step of setting a respective target region (S301) in a part of each ultrasonic image being processed so as to include a boundary extracted in an ultrasonic image preceding the an ultrasonic image being processed; and
a step of performing boundary extraction processing (S303) in the ultrasonic image being processed by using received signals corresponding to reflection waves reflected at an inner side of the target region thus set.

10. The control method according to claim 9, wherein
the time series ultrasonic images are respectively generated at individual positions of the ultrasonic probe on the object to be inspected; and
the target region is set in a part of the ultrasonic image being processed based on information of movement between the positions of the ultrasonic probe.

## Patentansprüche

1. Ultraschallgerät, mit:
einer Ultraschallsonde (10), die Ultraschallwellen an ein Inneres eines zu untersuchenden Objekts sendet, und von diesem Reflexionswellen empfängt;
einer Empfangseinheit (14), die basierend auf den von der Ultraschallsonde empfangenen Reflexionswellen empfangene Signale ausgibt;
einer Bildgenerierungseinheit (15, 24), die aus den empfangenen Signalen Ultraschallbilder erzeugt;
einer Grenzenextraktionseinheit (2), die basierend auf der Intensität jedes der empfangenen Signale die Grenzen zwischen in jedem der Ultraschallbilder vorkommenden Medien extrahiert;
einer Anzeigeeinheit (16); und
wobei die Grenzenextraktionseinheit eine Grenzenextraktionsverarbeitung bei einzelnen Ultraschallbildern einer Zeitreihe, die sequentiell erzeugt werden, durchführt,
**dadurch gekennzeichnet, dass** die Grenzenextraktionseinheit
einen jeweiligen Zielbereich in einem Teil von jedem verarbeitet werdenden Ultraschallbild derart einstellt, dass Grenzen umfasst sind, die in einem dem verarbeitet werdenden Ultraschallbild vorausgehenden Ultraschallbild extrahiert wurden, und
eine Grenzenextraktionsverarbeitung in dem verarbeitet werdenden Ultraschallbild durchführt, indem empfangene Signale verwendet werden, die Reflexionswellen entsprechen, die an einer Innenseite des somit eingestellten Zielbereichs reflektiert werden.

2. Ultraschallgerät gemäß Anspruch 1, wobei die Ultraschallbilder einer Zeitreihe jeweils bei einzelnen Positionen der Ultraschallsonde auf dem zu untersuchenden Objekt erzeugt werden; und
der Zielbereich in einem Teil des verarbeitet werdenden Ultraschallbilds eingestellt ist, basierend auf Bewegungsinformationen zwischen den Positionen der Ultraschallsonde.

3. Ultraschallgerät gemäß Anspruch 1, des Weiteren mit:
eine Anfangsgrenzeneinstellungseinheit, die eine Anfangsgrenze für ein erstes Ultraschallbild der Ultraschallbilder einer Zeitreihe einstellt.

4. Ultraschallgerät gemäß Anspruch 3, wobei
die Anzeigeeinheit das durch die Bilderzeugungseinheit produzierte Ultraschallbild anzeigt, und
die Anfangsgrenzeneinstellungseinheit die Anfangsgrenzen erfasst, entweder basierend auf Grenzenkandidatenpunkten, die auf dem auf der Anzeigeeinheit gezeigten ersten Ultraschallbild markiert sind, oder durch die Verwendung eines Bereichs von Interesse, der auf dem auf der Anzeigeeinheit gezeigten ersten Ultraschallbildausgewählt ist.

5. Ultraschallgerät gemäß Anspruch 1, des Weiteren mit:
einer Schallgeschwindigkeitseinstellungseinheit, die einen Wert einer Schallgeschwindigkeit für jeden von den Grenzen begrenzten Bereich einstellt.

6. Ultraschallgerät gemäß Anspruch 5, wobei
die Schallgeschwindigkeitseinstellungseinheit den Wert der Schallgeschwindigkeit für jeden Bereich bestimmt, gemäß einem Gewebenamen, der in jedem von den Grenzen begrenzten Bereich eingestellt ist.

7. Ultraschallgerät gemäß Anspruch 5, des Weiteren mit,
einer Bildverarbeitungseinheit, die das Ultraschallbild anhand der Verwendung des in jedem Bereich durch die Schallgeschwindigkeitseinstellungseinheit eingestellten Wertes der Schallgeschwindigkeit korrigiert.

8. Ultraschallgerät gemäß Anspruch 5, wobei
die Ultraschallsonde eine Vielzahl von Ultraschallwandlern aufweist;
das Gerät eine Phasenanpassungsberechnungseinheit umfasst, die die empfangenen Signale nach dem, entsprechend einer Fokusposition zu den empfangenen Signalen der einzelnen Ultraschallwandlern, jeweiligen Hinzufügen von Verzögerungen aufsummiert; und
die Phasenanpassungsberechnungseinheit durch die Verwendung des Wertes der Schallgeschwindigkeit in jedem durch die Schallgeschwindigkeitseinstellungseinheit eingestellten Bereich eine Verzögerungszeit von jedem Ultraschallwandler berechnet.

9. Steuerungsverfahren für ein Ultraschallgerät, das eine Ultraschallsonde (10), die Ultraschallwellen an ein Inneres eines zu untersuchenden Objekts sendet, und von diesem Reflexionswellen empfängt, eine Empfangseinheit (14), die basierend auf den von der Ultraschallsonde empfangenen Reflexionswellen empfangene Signale ausgibt, eine Bildgenerierungseinheit (15, 24), die aus den empfangenen Signalen Ultraschallbilder erzeugt, und eine Grenzenextraktionseinheit (2), die basierend auf der Intensität jedes der empfangenen Signale die Grenzen zwischen in jedem der Ultraschallbilder vorkommenden Medien extrahiert, und einer Anzeigeeinheit (16) umfasst,
wobei das Steuerungsverfahren für ein Ultraschallgerät aufweist:
einen Schritt des sequentiellen Generierens von Ultraschallbildern einer Zeitreihe aus den empfangenen Signalen; und
einen Schritt des sequentiellen Grenzenextrahierens hinsichtlich des dadurch produzierten Ultraschallbilds;
**dadurch gekennzeichnet, dass** der Schritt des Extrahierens der Grenzen umfasst:
einen Schritt des derartigen Einstellens eines jeweiligen Zielbereichs (S301) in einem Teil von jedem verarbeitet werdenden Ultraschallbild, dass Grenzen umfasst werden, die in einem dem verarbeitet werdenden Ultraschallbild vorausgehenden Ultraschallbild extrahiert wurden, und
einen Schritt des Durchführens einer Grenzenextraktionsverarbeitung (S303) in dem verarbeitet werdenden Ultraschallbild, durch das Verwenden empfangener Signale, die Reflexionswellen entsprechen, die an einer Innenseite des somit eingestellten Zielbereichs reflektiert werden.

10. Steuerungsverfahren gemäß Anspruch 9, wobei
die Ultraschallbilder einer Zeitreihe jeweils bei einzelnen Positionen der Ultraschallsonde auf dem zu untersuchenden Objekt erzeugt werden; und
der Zielbereich in einem Teil des verarbeitet werdenden Ultraschallbilds eingestellt wird, basierend auf Bewegungsinformationen zwischen den Positionen der Ultraschallsonde.

## Revendications

1. Appareil ultrasonore, comprenant :
une sonde ultrasonore (10) qui émet des ondes ultrasonores vers une partie intérieure d'un objet à inspecter, et qui en reçoit des ondes de réflexion ;
une unité de réception (14) qui délivre des signaux reçus sur la base des ondes de réflexion reçues par la sonde ultrasonore ;
une unité de génération d'images (15, 24) qui produit des images ultrasonores à partir des signaux reçus ;
une unité d'extraction de limites (2) qui extrait des limites entre des milieux apparaissant dans chacune des images ultrasonores sur la base de l'intensité de chacun des signaux reçus ;
une unité d'affichage (16) ; et
dans lequel l'unité d'extraction de limites exécute un traitement d'extraction de limites pour des images ultrasonores chronologiques individuelles générées successivement,
**caractérisé en ce que** l'unité d'extraction de limites
définit une région cible respective dans une partie de chaque image ultrasonore en cours de traitement pour qu'elle comprenne des limites extraites dans une image ultrasonore précédant l'image ultrasonore en cours de traitement, et
exécute un traitement d'extraction de limites dans l'image ultrasonore en cours de traitement au moyen de signaux reçus correspondant à des ondes de réflexion réfléchies au niveau d'un côté interne de la région cible ainsi définie.

2. Appareil ultrasonore selon la revendication 1, dans lequel
les images ultrasonores chronologiques sont respectivement générées à des positions individuelles de la sonde ultrasonore sur l'objet à inspecter ; et
la région cible est définie dans une partie de l'image ultrasonore en cours de traitement sur la base d'informations de déplacement entre les positions de la sonde ultrasonore.

3. Appareil ultrasonore selon la revendication 1, comprenant en outre :
une unité de définition de limite initiale qui définit une limite initiale pour une première image ultrasonore des images ultrasonores chronologiques.

4. Appareil ultrasonore selon la revendication 3, dans lequel
l'unité d'affichage affiche l'image ultrasonore produite par l'unité de génération d'images, et
l'unité de définition de limite initiale détecte la limite initiale soit sur la base de points candidats de limite marqués sur la première image ultrasonore affichée sur l'unité d'affichage soit au moyen d'une région d'intérêt sélectionnée sur la première image ultrasonore affichée sur l'unité d'affichage.

5. Appareil ultrasonore selon la revendication 1, comprenant en outre :
une unité de définition de vitesse de son qui définit une valeur d'une vitesse de son dans chacune de régions délimitées par les limites.

6. Appareil ultrasonore selon la revendication 5, dans lequel
l'unité de définition de vitesse de son décide de la valeur de la vitesse de son dans chaque région conformément à un nom de tissu défini dans chacune des régions délimitées par les limites.

7. Appareil ultrasonore selon la revendication 5, comprenant en outre :
une unité de traitement d'image qui corrige l'image ultrasonore au moyen de la valeur de la vitesse de son dans chaque région définie par l'unité de définition de vitesse de son.

8. Appareil ultrasonore selon la revendication 5, dans lequel
la sonde ultrasonore comporte une pluralité de transducteurs ultrasonores ;
l'appareil comprend une unité de calcul d'adaptation de phase qui additionne les uns aux autres les signaux reçus après avoir appliqué des retards correspondant à une position de foyer aux signaux reçus respectivement des transducteurs ultrasonores individuels ; et
l'unité de calcul d'adaptation de phase calcule un retard temporel de chaque transducteur ultrasonore au moyen de la valeur de la vitesse de son dans chaque région définie par l'unité de définition de vitesse de son.

9. Procédé de commande d'un appareil ultrasonore qui comprend une sonde ultrasonore (10) qui émet des ondes ultrasonores vers une partie intérieure d'un objet à inspecter, et qui en reçoit des ondes de réflexion, une unité de réception (14) qui délivre des signaux reçus sur la base des ondes de réflexion reçues par la sonde ultrasonore, une unité de génération d'images (15, 24) qui génère des images ultrasonores à partir des signaux reçus, et une unité d'extraction de limites (2) qui extrait des limites entre des milieux apparaissant dans chacune des images ultrasonores sur la base de l'intensité de chacun des signaux reçus, et une unité d'affichage (16),
le procédé de commande d'un appareil ultrasonore comprenant :
une étape consistant à générer successivement des images ultrasonores chronologiques à partir des signaux reçus ; et
une étape consistant à extraire successivement des limites par rapport aux images ultrasonores ainsi produites ;
**caractérisé en ce que** l'étape d'extraction des limites comprend :
une étape consistant à définir une région cible respective (S301) dans une partie de chaque image ultrasonore en cours de traitement pour qu'elle comprenne une limite extraite dans une image ultrasonore précédant l'image ultrasonore en cours de traitement ; et
une étape consistant à exécuter un traitement d'extraction de limites (S303) dans l'image ultrasonore en cours de traitement au moyen de signaux reçus correspondant à des ondes de réflexion réfléchies au niveau d'un côté intérieur de la région cible ainsi définie.

10. Procédé de commande selon la revendication 9, dans lequel
les images ultrasonores chronologiques sont générées respectivement à des positions individuelles de la sonde ultrasonore sur l'objet à inspecter ; et
la région cible est définie dans une partie de l'image ultrasonore en cours de traitement sur la base d'informations de déplacement entre les positions de la sonde ultrasonore.
